# EUROPEAN PATENT APPLICATION

(11) **EP 2 359 814 A1**
(43) Date of publication of application: **24.08.2011**
(21) Application number: 10382031.2
(22) Date of filing: 11.02.2010
(51) Int. Cl.: A61K 9/20, A61K 9/28, A61K 9/48, A61K 31/445

(54) **Pharmaceutical mini-tablets for sustained release of flecainide acetate**

(71) Applicant: Laboratorios Liconsa, S.A., 08028 Barcelona (ES)
(72) Inventor: Arroyo Hidalgo, Sergio, 08028, Barcelona (ES); Rizo Martinez, José Miguel, 08028, Barcelona (ES); Castilla, Teresa, 08028, Barcelona (ES)
(74) Representative: Ponti Sales, Adelaida

(57) **Abstract**

The invention relates to a pharmaceutical mini-tablet for sustained release of flecainide acetate which exhibits a different sustained profile, comprising a core including flecainide acetate, microcrystalline cellulose, and a disintegrant, and an outer cover layer including a pH-dependent release polymer and excipients.

Also relates to a process for the preparation of said mini-tablet which comprises a) blending flecainide acetate with microcrystalline cellulose and disintegrant to obtain a blended mixture; b) granulating the blended mixture with an aqueous solution of a binder; c) drying the obtained granulate; d) lubricating, when present, the dried granules and compressing it to obtain the core; and e) preparing an aqueous dispersion which comprises the pH-dependent polymer with excipients and coating the core to obtain the outer cover layer.

Thus, it provides an efficacious sustained release of flecainide acetate in a pharmaceutical form for oral administration in the treatment of cardiac arrhythmias.

## Description

### Field of the invention

The present invention relates to a pharmaceutical mini-tablet for sustained release of flecainide acetate which exhibits a different sustained profile, said mini-tablets comprising a core including flecainide acetate, microcrystalline cellulose, and a disintegrant, and an outer cover layer including a pH-dependent release polymer and one or more pharmaceutically acceptable excipients.

### Background of the invention

Flecainide acetate is an antiarrhythmic agent used to prevent and treat tachyarrhythmias (abnormal fast rhythms of the heart). It is used to treat cardiac arrhythmias including paroxysmal atrial fibrillation (episodic irregular heartbeat originating in the upper chamber of the heart), paroxysmal supraventricular tachycardia (episodic rapid but regular heartbeat originating in the atrium), and ventricular tachycardia (rapid rhythms of the lower chambers of the heart). Flecainide works by regulating the flow of sodium in the heart, causing prolongation of the cardiac action potential.

The development of efficacious modified release composition of flecainide acetate is hampered by the fact that this type of composition usually contains higher concentration of the active ingredient when compared with immediate release compositions on the same active.

EP 371683-A discloses a flecainide acetate controlled release pharmaceutical formulation comprising a film-coated bead. The bead comprises 75 percent by weight based on the total weight of the bead of flecainide acetate and 25 percent by weight of microcrystalline cellulose. The film-coat comprises 55.7 percent by weight based on the total weight of the film-coat of a methacrylic acid/methyl methacrylate copolymer (Eudragit), 33.3 percent by weight of talc and 10 percent by weight of polyethylene glycol.

The bead is prepared by granulating a mixture of flecainide acetate and microcrystalline cellulose using water as a granulating liquid. It is currently preferred that the flecainide acetate be milled through a 40 mesh screen and the microcrystalline cellulose be sifted through a 500 micrometer sieve prior to granulation. The granulate is then put through an extruder head and the extrudate is in turn placed in a spheroniser. The resulting spheres are dried until they have a moisture content of approximately 1% by weight. The dried spheres are sifted to remove both oversize beads and fines. A suspension suitable for film-coating the beads is prepared by dispersing first the talc and then the polyethylene glycol in a solution of the copolymer in a mixture of water and ethanol. The film-coating suspension is applied to the beads using a conventional fluidized-bed system

The total yield of an extrusion-spheronization method will depend upon many factors. On the one hand, during the extrusion phase it is essential to control dimensions such as the cross-section and the length of the extrudate so as to avoid great dispersion in the size and shape of the particles. Both factors would result in the subsequent coating being irregular and would even lead to the presence of pores, unless an excess quantity were projected in order to ensure complete coating of the microgranule, though this would in turn cause problems when it came to standardizing release of the active ingredient. On the other hand, the properties of cohesiveness, firmness and plasticity of the extrudate must be controlled if its subsequent spheronization is to be ensured.

To these problems it is also added the need to use several pieces of equipment such as kneading machines, extruding machines and spheronizers means that losses through kneading, extrusion and spheronization can be greater than with other pelletization methods.

Further, the flecainide acetate controlled release formulations would be released at about the time that the dosage form reaches the inlet between the small intestine and the colon, or thereafter in the colon.

Therefore, there is a need to provide a pharmaceutical formulation of flecainide acetate which exhibits an adequate release of flecainide acetate and a process for obtaining it in a good yield.

### Brief description of the invention

On the basis of numerous studies, the present inventors have found that is possible to provide an efficacious sustained release of flecainide acetate and a process that avoids the drawbacks of the prior art.

The present invention provides an adequate release multiparticulate (MP) delivery system of flecainide acetate in the form of mini-tablets. The plurality of mini-tablets according to the invention may be contained in a capsule or a sachet for oral administration After ingestion, mini-tablets according to the invention are released in the stomach, predictably transit to the small intestine and spread along the gastro-intestinal tract resulting in a consistent drug release with reduced risk of local irritation.

The present inventors have surprisingly found that is possible to modify the release profile of flecainide acetate of the invention by incorporating a disintegrant in the core of the mini-tablet formulation.

The concentration of the disintegrant present in the core is selected in such a way to give sustained release of flecainide acetate in a predetermined manner.

The invention also provides a process for the preparation of said pharmaceutical mini-tablet designed for the sustained release of flecainide acetate which exhibit a different sustained release profile avoiding the drawbacks of the prior art.

Another aspect of the invention is to provide a pharmaceutical form consisting of one or more mini-tablets and contained in a sachet or a capsule for their administration once or twice daily dosage.

At least another object of the invention relates to the use of said pharmaceutical form for oral administration in the form of mini-tablets in the treatment of cardiac arrhythmias.

### Figure

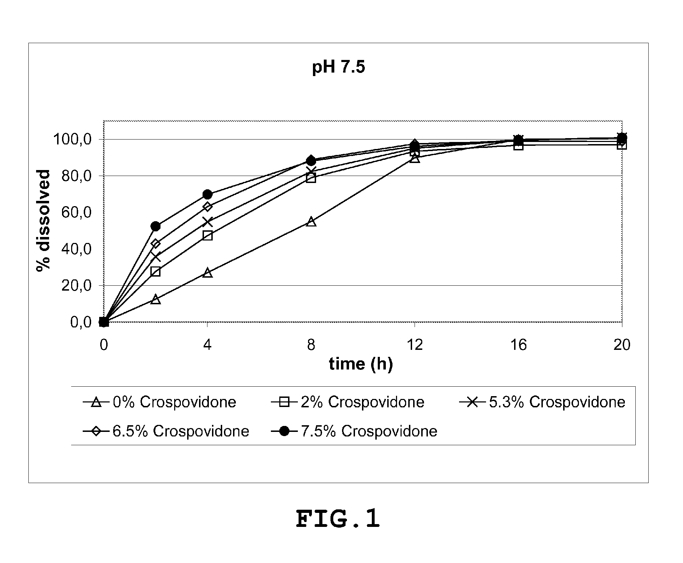

Figure 1 shows the dissolution profile obtained according to the pharmaceutical mini-tablet of the present invention.

### Detailed description of the invention

The present invention provides a pharmaceutical mini-tablet designed for the sustained release of flecainide acetate which exhibits a different sustained release profile, comprising a core and an outer cover layer, wherein:
said core comprises flecainide acetate, microcrystalline cellulose, and a disintegrant, and
said outer cover layer comprises a pH-dependent release polymer and one or more pharmaceutically acceptable excipients.

As used herein, "sustained release of flecainide acetate" means a dosage form in which the release of flecainide acetate is modified (or sustained) over a period of time compared to an immediate release formulation.

The preparation of the flecainide acetate does not form part of the object of the present invention and it may be carried out by any process disclosed in the state of the art as in patents US 4,339,587, US 4,497,954 or US 4,555,573.

According to the present invention, flecainide acetate may be in any crystalline or amorphous form.

The flecainide acetate is present from 50% to 90%, preferably from 60% to 80%, more preferably from 65% to 75% by weight based on the total weight of the mini-tablet.

Suitably, the microcrystalline cellulose may be present from 2% to 50%, preferably 5% to 40%, and more preferably from 8% to 30% by weight based on the total weight of the mini-tablet.

Suitably, the mini-tablet(s) comprises a binding agent or binder. The binder is selected from the group consisting of polyvinylpyrrolidone, hydroxypropylcellulose, hydroxypropylnethylcellulose, hydroxyethylcellulose, methylcellulose, sodium carboxymethyl cellulose, calcium carboxymethylcellulose, calcium carboxymethyl cellulose and/or mixtures thereof. Preferred binding agent is polyvinylpyrrolidone. The binder may be present in an amount from 0.5% to 10% by weight, preferably from 2% to 9% by weight, and more preferably from 2.5% to 7.5% by weight of the total weight of the mini-tablet.

Suitably, the mini-tablet formulation according to the invention optionally comprises an antiadherent agent in the core. The outer cover layer also may comprise an antiadherent agent to eliminate sticking during the film coating process.

Suitable antiadherent agents include colloidal silicon dioxide and talc, being preferable the colloidal silicon dioxide (aerosil) when is used in the core and talc when is used in the outer cover layer.

The antiadherent agent is present in the core in an amount of from 0.1% to 5% by weight, preferably from 0.1% to 3.5% by weight, more preferably from 0.1% to 1.5% by weight based on the total weight of the mini-tablet, and up to 70% by weight, preferably from 40% to 70% by weight based on the total weight of the outer cover layer.

Suitably, the mini-tablet formulation according to the invention optionally comprises a lubricant. Suitable lubricants include stearic acid, and stearic acid salts such as magnesium stearate. A preferred lubricant is magnesium stearate. The lubricant may be present 0.1% to 5% by weight, preferably from 0.1% to 3% by weight based on the total weight of the mini-tablet.

The mini-tablet(s) of the formulation are coated with one or more layers of a pH-dependent release polymer also named herein enteric polymer.

As used herein, the term "pH-dependent release polymer" means a polymer that is insoluble at the highly acidic pH found in the stomach, but dissolves rapidly at a less acidic (relatively more basic) pH. Thus, the pH-dependent release polymer will not dissolve in the acidic juices of the stomach (pH ~3), but will do it in the higher pH environment present in the small intestine as at a pH above 5.5 or in the colon as at a pH above 7.0.

The pH-dependent release polymer is selected such that flecainide acetate will be released at about the time that the dosage form reaches the inlet between the small intestine and the colon, or thereafter in the colon. The selection is based upon the pH profile of the small intestine and colon. The pH of the small intestine gradually increases from about 5 to 5.5 in the duodenal bulb to about 7.2 in the distal portions of the small intestine (ileum). The pH drops significantly at the ileocecal junction to about 6.3 and very gradually increases to about 7 in the left or descending colon.

With the pharmaceutical mini-tablet of the present invention it is possible to provide a colonic release for systemic absorption of flecainide acetate for which peak systemic concentrations and pharmacological activity are desired at time significantly delayed from the time of peroral administration.

Preferred pH-dependent release polymers are those, which remain intact in the lower pH environs of the stomach and small intestine, but begin to dissolve in an aqueous solution at a pH above 6.3, preferably between 6.8 to 7.2. Preferred enteric polymer are selected from poly(methacrylic acid, methyl methacrylate) 1:2 (Eudragit® S), and mixtures of poly(methacrylic acid, methyl methacrylate) 1:1 (Eudragit® L) and poly(methacrylic acid, methyl methacrylate) 1:2 (Eudragit® S) in a ratio of about 1:10 to about 1:1, preferably about 1:5 to about 1:3. Especially preferred is Eudragit® S.

In a preferred embodiment, the pH-dependent release polymer is present in an amount from 15% to 75% by weight of the outer cover layer of the mini-tablet.

Suitably, outer cover layer is present from 1% to 10% based on the total weight of the mini-tablet formulation, preferably from 2% to 5%.

The mini-tablets according to the present invention have a diameter of less than 5 mm and an outer cover layer thickness dependent upon the size of the mini-tablet(s), but ranging from 5 µm to 20 µm.

Suitably, the outer cover layer optionally comprises a plasticizer to provide homogeneous film mixtures. Preferred plasticizer includes triethyl citrate, polyethylene glycol, or dibutyl sebacate, preferably polyethylene glycol. The plasticizer agent may be present in an amount up to 20%, preferably from 5% to 20% y weight based on the total weight of the outer cover layer.

Although disintegrant agents are excipients that promote the rapid breakup of the formulation in an aqueous medium, as well as the rapid disintegration of the formulation in order to release more quickly the active substance, and therefore are not suitable for sustained release formulations, the present inventors have surprisingly found that its presence modifies the sustained release profile of flecainide acetate of mini-tablet formulation maintaining at the same time the sustained release character of the mini-tablet formulation. Varying the amount of disintegrant agent present in the core, the sustained release profile of the mini-tablet formulation can be modified in a predetermined manner. The authors have surprisingly found that the mini-tablet formulation of the invention can retain its sustained release profile at disintegrant concentrations up to 10% by weight based on the total weight of the mini-tablet. The best results were obtained at disintegrant concentrations from 5% to 7.5%.

Disintegrant agent may be selected from the group consisting of low-substituted hydroxypropylcellulose, sodium carboxymethyl cellulose, crospovidone, sodium croscarmellose and/or mixtures thereof. Preferably, the disintegrant agent is crospovidone.

The disintegrant agent is present in the core of the mini-tablet(s) from 2% to 10% by weight, preferably from 5% to 7.5% by weight based on the total weight of the mini-tablet.

The formulation of mini-tablet(s) of the invention includes one or more pharmaceutically acceptable excipients. All such excipients must be "pharmaceutically acceptable" in the sense of being compatible with the other ingredients of the pharmaceutical composition and not injurious to the patient. Pharmaceutically acceptable excipients may include colours, flavours e.g. menthol, sweeteners e.g. mannitol, preservatives, stabilisers, antioxidants and any other excipients known to those skilled in the art.

In a preferred embodiment, the pharmaceutical mini-tablet designed for the sustained release of flecainide acetate which exhibits a different sustained release profile comprises a core comprising from 60% to 80% by weight of flecainide acetate, from 5% to 40% of microcrystalline cellulose, from 2% to 9% by weight of polyvinyl pyrrolidone, from 2% to 9% by weight of crospovidone and, up to 10% by weight of a disintegrant, and the outer cover layer comprising a pH-dependent release polymer and one or more pharmaceutically acceptable excipients.

In a more preferred embodiment, the pharmaceutical mini-tablet designed for the sustained release of flecainide acetate comprises a core comprising from 65% to 75% of flecainide acetate, from 8% to 30% of microcrystalline cellulose, from 2.5% to 7.5% by weight of polyvinyl pyrrolidone, and from 2% to 10% of a disintegrant, and an outer cover layer comprising a pH dependent release polymer and one or more pharmaceutically acceptable excipients.

In a still more preferred embodiment, the pharmaceutical mini-tablet designed for the sustained release of flecainide acetate comprises a core comprising from 65% to 75% of flecainide acetate, from 8% to 30% of microcrystalline cellulose, from 2.5% to 7.5% by weight of polyvinyl pyrrolidone and from 5% to 7.5% by weight of a disintegrant, and an outer cover layer comprising a pH-dependent release polymer and one or more pharmaceutically acceptable excipients

Preferably the disintegrant agent is crospovidone, and the pH dependent polymer is poly(methacrylic acid, methyl methacrylate) 1:2 (Eudragit (® S).

Advantageously, the pharmaceutical mini-tablet according to the invention has a diameter of less than 5 mm and the outer cover layer has a thickness ranging from 5 µm to 20 µm.

The second aspect of the present invention is to provide a process for the preparation of the pharmaceutical mini-tablet designed for the sustained release of flecainide acetate which exhibits a different sustained release profile according to the invention, said process comprising the steps of:
a) blending the flecainide acetate with the microcrystalline cellulose and the disintegrant to obtain a blended mixture;
b) granulating the blended mixture with an aqueous solution of binder, previously prepared;
c) drying the granulate obtained in step b);
d) lubricating, if a lubricant is present, the dried granules and compressing it to obtain the core; and
e) preparing an aqueous dispersion which comprises the pH-dependent polymer with the pharmaceutically acceptable excipient and coating the core to obtain the outer cover layer, and thus the mini-tablet.

The method used for coating the mini-tablet(s) may be any conventional methods known to a person skilled in the art.

Advantageously, in step a) an antiadherent agent is added; in step b) an aqueous solution of polyvinyl pyrrolidone solution at 15% in water is prepared; in step c) the drying is carried out in a fluid bed dryer; in step d) a lubricant is added for lubricating the dried granules and, then, the blend compressed to obtain a core diameter of less than 5 mm; and in step e) an aqueous dispersion of an alcoholic solution of an antiadherent agent and the pH-dependent polymer and a plastisizer is prepared for coating the core obtaining an outer cover layer of a thickness ranging from 5 µm to 20 µm.

The present invention also relates to a pharmaceutical form consisting of one or more mini-tablets according to the first aspect of the invention, and contained in a sachet or a capsule to provide a vehicle for a quantity of flecainide acetate corresponding to once or twice daily dose. Preferably, the capsule is a hard gelatin or hydroxymethylcellulose (HPMC) capsule.

Mini-tablets according to the invention can also be compacted into larger tablets which, after disintegration, release the sub-units as a multiparticulate system. In a preferred embodiment, mini-tablet(s) according to the invention are contained in a capsule for oral administration.

Further, flecainide acetate may be present in an amount of 25 to 300 mg per capsule. In a preferred embodiment, flecainide acetate is present in an amount of 50, 100, 150 or 200 mg per capsule.

Advantageously, the pharmaceutical form consists of a plurality of 6, 12, 24, 36, 48 or 60 mini-tablets. In a preferred embodiment, the pharmaceutical form of the invention comprises a plurality of 12, 24, 36 or 48 mini-tablets.

In another aspect, the present invention relates to the use of the pharmaceutical form for oral administration in the form of mini-tablets in the treatment of cardiac arrhythmias.

The use of said pharmaceutical form provides therapeutically effective levels of flecainide acetate over extended periods of time after oral administration, e.g. for at least 12 or 24 hours, thus enabling twice daily dosing or once daily dosing.

The following examples are given to illustrate the invention in a sufficiently complete manner.

### EXAMPLES

### Example 1

| **Tablet component** | **(mg/comp.)** | **Function** |
|---|---|---|
| **Tablet Core** | | |
| Flecainide acetate | 4,17 | API |
| Polyvinyl pyrrolidone (Povidone K-25) | 0,35 | Binder |
| Microcrystalline cellulose PH 102 | 0,82 | Diluent |
| Crospovidone | 0,32 | Disintegrant |
| Colloidal silicon dioxide (Aerosil) | 0,06 | Glidant |
| Magnesium stearate | 0, 12 | Lubricant |
| **Tablet coating** | | |
| Talc | 0, 12 | Glidant |
| Methacrylic acid/methyl methacrylate copolymer 1:2 (Eudragit® S) | 0,04 | Controlled Release Polymer |
| Polyethylene glycol (PEG 400) | 0,02 | Plasticizer |
| Ethyl alcohol* | 1, 63 | Solvent |
| Coating weight (dry) | 0, 18 | --- |
| **Coated tablet weight** | **6,02** | |

| | | |
|---|---|---|
| * It's evaporated during the process | | |

Flecainide acetate, microcrystalline cellulose, crospovidone and colloidal silicon dioxide (aerosil) are mixed and subsequently granulated with a polyvinyl pyrrolidone solution (15%) in purified water.

The granules are dried in a fluid bed dryer. The dried granules are passed through suitable mesh sieve and these granules and lubricated with magnesium stearate.

The final blend is then compressed in 2 mm diameter punches to obtain tablets of 5,84 mg weight (each microtablet contains 4,17 mg of flecainide acetate).

The tablets are coated with the gastro-resistant coating suspension (alcoholic solution of talc, Eudragit S 100 and PEG 400). The diameter of film coated mini-tablets ranges between 2 mm to 5 mm.

These mini-tablets are then filled into a hard gelatin capsule to form 200 mg strength of flecainide acetate.

### Examples 2 to 5

Using a procedure similar to that described in Example 1, mini-tablets containing 4,17 mg of flecainide acetate and the different amounts of crospovidone shown in Table 1 were prepared.

| Example | Disintegrant amount (% by weight)* |
|---|---|
| 2 | 0 |
| 3 | 2 |
| 4 | 6.5 |
| 5 | 7.5 |

| | |
|---|---|
| * The weight percent refers to the total weight of the mini-tablet(s). | |

These mini-tablets are then filled into a hard gelatin capsule to form 200 mg strength of flecainide acetate.

### Dissolution method

For all examples, the capsule containing the mini-tablets was tested for dissolution of flecainide acetate at buffer Trishydroximethylaminomethane pH 7.5, Apparatus 1, 100 rpm.

The dissolution profile obtained for the formulations described in examples 1 to 5 is disclosed in Figure 1.

## Claims

1. A pharmaceutical mini-tablet designed for the sustained release of flecainide acetate which exhibits a different sustained release profile, comprising a core and an outer cover layer, wherein:
said core comprises flecainide acetate, microcrystalline cellulose, and a disintegrant, and
said outer cover layer comprises a pH-dependent release polymer and one or more pharmaceutically acceptable excipients.

2. A pharmaceutical mini-tablet according to claim 1, wherein the flecainide acetate is present from 50% to 90%, preferably from 60% to 80%, more preferably from 65% to 75% by weight based on the total weight of the mini-tablet.

3. A pharmaceutical mini-tablet according to claim 1, wherein the microcrystalline cellulose is present from 2% to 50%, preferably from 5% to 40%, and more preferably from 8% to 30% by weight based on the total weight of the mini-tablet.

4. A pharmaceutical mini-tablet according to claim 1, wherein the disintegrant is selected from the group consisting of low-substituted hydroxypropylcellulose, sodium carboxymethyl cellulose, crospovidone, sodium croscarmellose and/or mixtures thereof, preferably crospovidone.

5. A pharmaceutical mini-tablet according to claim 1, wherein the disintegrant is present up to 10% by weight, preferably form 2% to 10% by weight, more preferably from 5% to 7.5% by weight based on the total weight of the mini-tablet.

6. A pharmaceutical mini-tablet according to claim 1, wherein the core further comprises at least one of the following: a binder, an antiadherent agent, or a lubricant.

7. A pharmaceutical mini-tablet according to claim 6, wherein the binder is selected from the group consisting of polyvinylpyrrolidone, hydroxypropylcellulose, hydroxypropylnethylcellulose, hydroxyethylcellulose, methylcellulose, sodium carboxymethyl cellulose, calcium carboxymethylcellulose, calcium carboxymethyl cellulose and/or mixtures thereof.

8. A pharmaceutical mini-tablet according to claim 6, wherein the binder is present from 0.5% to 10% by weight, preferably from 2% to 9% by weight, and more preferably from 2.5% to 7.5% by weight based on the total weight of the mini-tablet.

9. A pharmaceutical mini-tablet according to claim 6, wherein the antiadherent is selected from colloidal silicon dioxide and talc, and is present from 0.1% to 5% by weight, preferably from 0.1% to 3.5% by weight, more preferably from 0.1% to 1.5% by weight based on the total weight of the mini-tablet.

10. A pharmaceutical mini-tablet according to claim 6, wherein the lubricant is selected from stearic acid, and stearic acid salts, preferably magnesium stearate, and is present in an amount from 0.1% to 5% by weight, preferably from 0.1% to 3% by weight based on the total weight of the mini-tablet.

11. A pharmaceutical mini-tablet according to claim 1, wherein the pH-dependent release polymer is selected from poly(methacrylic acid, methyl methacrylate) 1:2, and mixtures of poly(methacrylic acid, methyl methacrylate) 1:1 and poly(methacrylic acid, methyl methacrylate) 1:2 in a ratio from 1:10 to 1:1, preferably from 1:5 to 1:3.

12. A pharmaceutical mini-tablet according to claim 1, wherein the outer cover layer is present from 1% to 10% by weight, preferably from 2% to 5% by weight based on the total weight of the mini-tablet.

13. A pharmaceutical mini-tablet according to claim 1, wherein the pH-dependent release polymer is present from 15% to 75% by weight based on the total weight of the outer cover layer.

14. A pharmaceutical mini-tablet according to claim 1, wherein the outer cover layer further comprises at least one of the followings: an antiadherent agent or a plastisizer.

15. A pharmaceutical mini-tablet according to claim 14, wherein the antiadherent agent is present up to 70% by weight, preferably from 40% to 70% by weight based on the weight of the outer cover layer.

16. A pharmaceutical mini-tablet according to claim 14, wherein the plastisizer is selected from triethyl citrate, polyethylene glycol, and dibutyl sebacate, preferably polyethylene glycol.

17. A pharmaceutical mini-tablet according to claim 14, wherein the plastisizer is present up to 20%, preferably from 5 to 20% by weight based on the total weight of the outer cover layer.

18. A pharmaceutical mini-tablet according to claim 1, wherein said mini-tablet has a diameter of less than 5 mm and the outer cover layer has a thickness ranging from 5 µm to 20 µm.

19. A process for the preparation of a pharmaceutical mini-tablet comprising a core and an outer cover layer according to any of claims 1 to 18, said process comprising the steps of:
a) blending the flecainide acetate with the microcrystalline cellulose and the disintegrant to obtain a blended mixture;
b) granulating the blended mixture with an aqueous solution of binder, previously prepared;
c) drying the granulate obtained in step b);
d) lubricating, if a lubricant is present, the dried granules and compressing it to form the core; and
e) preparing an aqueous dispersion which comprises the pH-dependent polymer with the pharmaceutically acceptable excipient and coating the core to form the outer cover layer, and thus the mini-tablet.

20. A process according to claim 19, wherein at least:
- in step a) an antiadherent agent is added;
- in step b) an aqueous solution of polyvinyl pyrrolidone solution at 15% in water is prepared;
- in step c) the drying is carried out in a fluid bed dryer;
- in step d) a lubricant is added for lubricating the dried granules and, then, the blend compressed to obtain a core diameter of less than 5 mm; and
- in step e) an aqueous dispersion of an alcoholic solution of an antiadherent agent and the pH-dependent polymer and a plastisizer is prepared for coating the core to obtain an outer cover layer thickness ranging from 5 µm to 20 µm.

21. A pharmaceutical form consisting of one or more mini-tablets according to any claims 1 to 18, and contained in a sachet or a capsule to provide a vehicle for a quantity of flecainide acetate corresponding to once or twice daily dose.

22. A pharmaceutical form according to claim 21, wherein the capsule is a hard gelatin or hydroxymethylcellulose (HPMC) capsule.

23. Use of a pharmaceutical form according to claim 21 for oral administration in the form of mini-tablets in the treatment of cardiac arrhythmias.
